# EUROPÄISCHE PATENTSCHRIFT

(11) **EP 0 402 922 B1**
(45) Veröffentlichungstag und Bekanntmachung des Hinweises auf die Patenterteilung: **27.03.1996**
(21) Anmeldenummer: 90111243.3
(22) Anmeldetag: 13.06.1990
(51) Int. Cl.: A61L 2/02

(54) **Verfahren zur Sterilisation von chirurgischem Material**
Process for the sterilization of a surgical material
Procédé pour la stériliation de matérieaux chirurgicaux

(30) Priorität: 14.06.1989 DE 3919391
(43) Veröffentlichungstag der Anmeldung: 19.12.1990
(73) Patentinhaber: Universität Karlsruhe (FH)/Technologie-Lizenz-Büro der Baden-Württembergischen Hochschulen, 76131 Karlsruhe (DE)
(72) Erfinder: Ludwig, Horst, Dr., Prof, D-6906 Leimen (DE); Stricker, Herbert, Dr., Prof., D-6903 Neckargemünd (DE); Entenmann, Günther, Dr., D-6507 Ingelheim am Rhein (DE)

(56) Entgegenhaltungen:
- FR-A- 2 442 018
- GB-A- 1 188 753
- LU-A- 71 446

## Beschreibung

Die Erfindung betrifft ein Verfahren zur Sterilisation von chirurgischen Materialien, insbesondere von Formkörpern aus einem biologisch abbaubaren Polymer.

In den letzten Jahren hat das Interesse an der Verwendung resorbierbarer Polymere - insbesondere von resorbierbaren Polyestern - in der Medizin sehr stark zugenommen. Die Herstellung und die Verwendung solcher Polymere ist aus dem Stand der Technik bekannt, so z.B. aus den DE-OS'en 12 93 396, 15 95 085, 17 20 211, 21 18 127, 22 06 144, 25 01 448, 27 00 729, 28 25 911, 27 27 289, 28 50 824, den europäischen Patentanmeldungen 73 655, 98 394, 102 265, 108 933, 176 895, 311 065, 58 481 u.a..

Solche Polymere finden im Bereich der Chirurgie Verwendung als Nahtmaterial, als Klammern, zum Wundverschluß im Bereich der Osteosynthese als Ersatz für Metallimplantate und als Wirkstoff-Freigabesysteme. Eine essentielle Voraussetzung für die Verwendung solcher biologisch abbaubarer Polymere ist, daß die aus diesen Polymeren hergestellten chirurgischen Materialien nur in steriler Form eingesetzt werden. Aufgrund der Stoffeigenschaften der biologisch abbaubaren Polymere sind die üblicherweise in der Medizin angewandten Sterilisationsmethoden nur beschränkt anwendbar. Die thermische Sterilisation kann nicht angewendet werden, da die hierzu notwendigen Temperaturen (größer 100°C) in vielen Fällen bereits im Schmelzbereich der biologisch abbaubaren Polymeren liegen. Eine andere - ebenfalls gebräuchliche - Methode zur Sterilisation unter Anwendung von γ-Strahlen kann bei Materialien aus biologisch abbaubaren Polymeren deshalb nicht angewendet werden, da durch die Bestrahlung das Polymer z.T. bereits abgebaut und die Abbaurate des Polymeren nach der Implantation in den Organismus ungünstig beeinflußt wird.

Aus dem Stand der Technik ist weiterhin ein Verfahren zur Behandlung medizinischer Gegenstände bekannt, bei dem die Gegenstände - wie z.B. Nahtmaterial - unter Zuhilfenahme eines Fluids hohen Drücken ausgesetzt werden [GB 1 188 753]. Bei diesem Verfahren werden die Keime jedoch nicht abgetötet, sondern für andere Sterilisationsmaßnahmen wie z.B. für ionisierende Strahlung, UV-Strahlung, Behandlung mit chemischen Agenzien oder Erhitzen sensibilisiert. Eine Abtötung der Keime allein durch Anwendung hoher Drucke kann mit Hilfe des dort beschriebenen Verfahrens nicht erfolgen.

Daneben sind beispielsweise aus der luxemburgischen Offenlegungsschrift 71 446 sowie aus der französischen Offenlegungsschrift 2 442 018 Verfahren zur Druckbehandlung von Lebensmitteln bekannt.

Zum gegenwärtigen Zeitpunkt können Gegenstände aus biologisch abbaubaren Polymeren nur durch die Anwendung bestimmter Gase - z.B. Ethylenoxid - sterilisiert werden. Dieses Verfahren weist verschiedene Nachteile auf: Ethylenoxid bildet mit Luft in weiten Grenzen explosive Gemische, reines Ethylenoxid zerfällt beim Erwärmen auch ohne Luft explosionsartig (aus diesen Gründen werden meistens Mischungen mit Inertgasen, wie CO₂ oder Freon, eingesetzt).
Ethylenoxid (EO) ist stark giftig (MAK in der BRD 10 ppm = 0,018 mg/l), reizt Atemwege und Haut und führt zu Kopfschmerzen und Erbrechen. 100-200 mg EO/l Atemluft sind für Menschen innerhalb weniger Sekunden tödlich. Außerdem wirkt Ethylenoxid mutagen beim Menschen und karzinogen im Tierversuch.
Ethylenoxid ist nur wirksam bei direktem Kontakt des Gases mit den Mikroorganismen (also nur an Oberfläche), außerdem ist Feuchtigkeit (Wasser) notwendig, da die Wirksamkeit stark von der Feuchte abhängt.

Es ist die Aufgabe der vorliegenden Erfindung, ein Verfahren vorzuschlagen, das eine einfache und sichere Sterilisation von Gegenständen aus einem biologisch abbaubaren Polymer ermöglicht.

Die Aufgabe wird erfindungsgemäß dadurch gelöst, daß die Gegenstände aus dem biologisch abbaubaren Polymer für eine vorherbestimmte Zeit hohen Drucken von mindestens 100 MPa = Mega-Pascal ausgesetzt werden. Bei diesem Verfahren ist es ausreichend, wenn Temperaturen in einem Bereich zwischen Raumtemperatur bis 90°C angewendet werden, bevorzugt im Bereich zwischen 40 und 60° C.

Im allgemeinen beträgt die aufzuwendende Zeit ca. 24 Stunden, sie kann jedoch - abhängig von der Verkeimung - auch nur wenige Stunden oder Minuten betragen. Die aufzuwendende Zeit ist druckabhängig, d.h. höhere Drücke von 150, 200, 250 oder 300 MPa verkürzen die Sterilisationszeit. Es empfiehlt sich den Enddruck in wenigen Minuten aufzubauen; ein sehr viel langsamerer Druckaufbau kann in manchen Fällen die Sterilisation nachteilig beeinflussen.
In einer Verfahrensvariante des erfindungsgemäßen Verfahrens werden die Formkörper einem geringeren Druck - d.h. zwischen 40 und 90 MPa ausgesetzt. Hierdurch beginnen eventuell vorhandene Sporen besonders gut zu keimen. Anschließend werden die Formkörper hohem Druck gemäß dem erfindungsgemäßen Verfahren ausgesetzt. Nach dieser Verfahrensvariante werden vorhandene Sporen von der Sterilisation mit umfaßt. Die Induzierung der Sporen kann sowohl zu Beginn wie auch im Verlauf des erfindungsgemäßen Verfahrens erfolgen.
Im einzelnen wird das erfindungsgemäße Verfahren wie folgt durchgeführt:
Der Formkörper kann zur Sterilisation unmittelbar den erforderlichen Drücken ausgesetzt werden. Hierzu bieten sich verschiedene Möglichkeiten an. Eine Variante besteht darin, daß der Formkörper in einem Autoklaven in Gegenwart eines inerten Gases den hohen Drücken ausgesetzt wird. Geeignete Gase sind solche, die weder mit dem Formkörper selbst - oder falls dieser einen Wirkstoff enthält - noch mit diesem Wirkstoff in unerwünschter Weise reagieren. Beispielsweise werden genannt: Luft, Stickstoff, Edelgase - wie z.B. Helium, gasförmige Halogenkohlenwasserstoffverbindungen, wie z.B. Freone.
Es ist denkbar, ein aus dem Stand der Technik bekanntes Gas oder Gasgemisch, das üblicherweise bei Normaldruck zur Sterilisation eingesetzt wird, bei dem erfindungsgemäßen Verfahren in geringer Konzentration einzusetzen, wie z.B. Ethylenoxid.

Für das erfindungsgemäße Verfahren können auch solche Gase oder Gasgemische eingesetzt werden, die unter den anzuwendenden Drücken von über 100 MPa in den flüssigen Aggregatzustand übergehen; ein Beispiel hierfür ist die Verwendung von CO₂.

In einer anderen Variante wird der Autoklav mit einer Flüssigkeit gefüllt, wodurch der Druck auf den Formkörper übertragen wird. Auch in diesem Fall sind solche Flüssigkeiten geeignet, deren direkter Kontakt mit dem Formkörper - diesen oder einen darin enthaltenen Wirkstoff nicht nachteilig beeinflußt. Geeignet sind beispielsweise, Wasser, organische Lösungsmittel - wie z.B. Kohlenwasserstoffe, Alkohole, Ketone, halogenierte Kohlenwasserstoffe, Freone, Glycerine, Öle, Silikonöl, physiologische Kochsalzlösung. Es sind auch Lösungsmittelgemische geeignet.
Die Wahl einer geeigneten Flüssigkeit kann von Fall zu Fall in Abhängigkeit des zu sterilisierenden Polymers und gegebenenfalls der chemischen Struktur eines Wirkstoffes unterschiedlich ausfallen. Es ist selbstverständlich, daß im Fall der beschriebenen Varianten nach Ende der Sterilisation beim Öffnen des Autoklaven Maßnahmen ergriffen werden müssen, damit die nunmehr sterilen Formkörper nicht erneut kontaminiert werden. Geeignete Maßnahmen hierzu sind an sich bekannt. Es wäre auch denkbar, daß für eine Realisierung im technischen Maßstab ein Autoklav mit zwei Öffnungen versehen ist, wobei die beiden Öffnungen in verschiedenen Räumen - einem nichtsterilen zum Beladen und einem sterilen zum Entladen und Verpacken der Formkörper - angeordnet sind.

In einer anderen erfindungsgemäßen Ausführungsform wird der Formkörper in einer Folie - z. B. in Form eines Polyethylen-Beutels - eingeschlossen.

Hierdurch wird sichergestellt, daß der Formkörper während der Druckbehandlung nicht mit den Flüssigkeiten oder Gasen des Autoklaven in Kontakt kommt. Ein weiterer Vorteil ist, daß nach Abschluß der Druckbehandlung keine besonderen Maßnahmen zur Verhinderung von Kontamination getroffen werden müssen. Der Formkörper kann in dieser Folie in trockenem oder feuchtem Zustand eingeschlossen sein. Entscheidend ist, daß der zur Sterilisation notwendige Druck auf den Formkörper übertragen werden kann. Wird eine Flüssigkeit mit dem Formkörper in der Folie eingeschlossen, so ist es sinnvoll nur solche Flüssigkeiten auszuwählen, die auch nach üblicher Lagerung keinen negativen Einfluß auf den Formkörper oder auf einen darin enthaltenen Wirkstoff ausüben. Die Benetzung der Oberfläche der Formkörper kann beispielsweise mit Wasser, physiologischer Kochsalzlösung, Glycerin, Glycolen, Oligoglycolen erfolgen. Es können auch zu einer Verbesserung der Benetzung pharmakologisch unbedenkliche oberflächenaktive Stoffe (Detergentien) eingesetzt werden.

In einer weiteren Ausführungsform der Erfindung wird der Beutel mit dem Formkörper vor der Sterilisation evakuiert. Um bei der Hochdrucksterilisation einen möglichst guten Kontakt zwischen dem Verpackungsmaterial und dem zu sterilisierenden Formkörper herzustellen, ist es sinnvoll, daß die Luft aus der Verpackung herausgesaugt wird, damit sich die Folie möglichst dicht an den Formkörper anschmiegt. Ein wesentlicher Vorteil dieser Ausführungsform ist, daß man - auch nach längerer Lagerung - anhand einer intakten Vakuumverpackung einfach feststellen kann, ob die Verpackung unversehrt und der Formkörper somit noch steril ist.

Wird die Sterilisation in einer Gasatmosphäre durchgeführt, ist es möglich, den Beutel mit einem bakteriendichten Filter zu versehen. Hierdurch kann beim Komprimieren die Luft aus dem verschlossenen Beutel entweichen; dies bewirkt einen besseren Kontakt zwischen Formkörper und Folie. Beim Belüften des Autoklaven können keine Bakterien eindringen; der Bakterienfilter kann anschließend abgeschmolzen werden.

In einer anderen erfindungsgemäßen Ausführungsform befindet sich der Formkörper in einer Glas- oder Kunststoffampulle, die nach der Drucksterilisation unter sterilen Bedingungen zugeschmolzen werden. Solche Ampullen können ebenfalls mit einem bakteriendichten Filter bei der Sterilisation versehen werden; hierdurch wird das Abschmelzen in einer nichtsterilen Umgebung ermöglicht.
Die erfindungsgemäßen Beutel (Verpackungen) können einen oder mehrere Formkörper enthalten. Eine größere Verpackung kann mehrere kleinere, separater Beutel mit je einem oder mehreren Formkörpern enthalten. Dies kann beispielsweise, dadurch erreicht werden, daß die Formkörper auf einer Folie ausgelegt werden und eine zweite Folie darübergelegt wird. Beide Folien werden nun so miteinander verbunden (verkleben, heißverschweißen), daß jeder einzelne Formkörper für sich vollständig abgeschlossen ist. Es können Vorrichtungen vorgesehen werden, z.B. eine Perforation, die es ermöglichen, die erhaltene Verpackung in separate Beutel mit je einem Formkörper abzutrennen.

Wie oben dargelegt, wird der Beutel vorzugsweise evakuiert und verschweißt. Er kann aber auch Restluft oder Inertgas wie Stickstoff, Edelgase u.a. enthalten, um eine Kontamination bei Beschädigung zu vermeiden.

Als Folien - bzw. Beutel - sind solche geeignet, die ausreichend elastisch sind, um sich an den Formkörper anschmiegen zu können, aber dennoch eine ausreichende mechanische Festigkeit aufweisen. Die Wahl bezüglich des Materials für die Folien ist nicht kritisch, soweit es pharmakologisch unbedenklich, bakteriendicht, bzw. undurchlässig für Mikroorganismen, wasserdicht, wasserdampfundurchlässig und mechanisch stabil ist. Geeignet sind beispielsweise Folien aus Polyethylen , Polypropylen, Polyamid, Polyurethan, Cellulose, Polyisobutylen, Polyacrylnitril, oder Polyvinylchlorid. Bevorzugt werden solche Folien, die keinen oder lediglich pharmakologisch unbedenkliche Weichmacher enthalten, wie z.B. Triethylcitrat (Trimethylcitrat), Tributylcitrat, Acetyltributylcitrat oder Triacetin.

Geeignete Folien weisen beispielsweise eine Stärke zwischen 0.01 mm und 0.5 mm auf. In Abhängigkeit der Reißfestigkeit und der Flexibilität des Folienmaterials können aber auch stärkere oder dünnere Folien verwendet werden, soweit sie die oben genannten Bedingungen erfüllen. Die Folien können auf ihrer Außenseite behandelt sein, so daß eine einfache - haltbare Beschriftung möglich ist. Weiterhin können die evakuierbaren Beutel Vorrichtungen enthalten, die ein einfaches Aufreißen des evakuierten Beutels - z.B. im Operationssaal - ermöglicht.

Erfindungsgemäß können nach dem oben beschriebenen Verfahren Formkörper aus jedem resorbierbaren, biologisch abbaubaren Polymer sterilisiert werden.

Das erfindungsgemäße Verfahren eignet sich auch zur Sterilisation von Formkörpern, die aus nichtresorbierbaren Materialien hergestellt sind; beispielsweise aus nichtresorbierbaren Polymeren, wie z.B. Polyurethan, Polyethylen, Polypropylen und Polyester oder anorganischen Materialien wie beispielsweise Tricalciumphosphat oder Hydroxylapatid.

Beispiele für solche Formkörper sind:
1. Massive Produkte, formgepreßt oder maschinell bearbeitet:
   Orthopädische Dübel, Klammern, Schrauben und Platten, Clips (z.B. für vena cava),
   Krampen,
   Haken, Knöpfe oder Schnapper,
   Knochenersatz (z.B. Kieferprothesen),
   Nadeln,
   nichtpermanente Intrauterineinsätze (antispermizid),
   Chirurgische Instrumente,
   Gefäßimplantate oder -stützmittel,
   Wirbelscheiben,
   Wirkstoffe freigebende Erzeugnisse (Pillen, Pellets),
   verstärkte Knochendübel, Nadeln usw.,
   implantierbare mit Arzneistoffen beladene Pellets, Stifte, Folien und sonstige Formkörper zur kontrollierten Wirkstofffreigabe.
2. Halbfeste oder flüssige Materialien, wie z.B. resorbierbare Knochenwachse, Knochen- und Gewebekleber, Knochenzemente, Füllmassen für die plastische Chirurgie oder die Zahnmedizin.
3. Als Wirkstoffträger geeignete Pulver Mikrokapseln oder Nanopartikel.
4. Gesponnene, Geflochtene und/oder gewobene Gegenstände wie chirurg. Nahtmaterial, Bänder, Kordeln, Netze, Vliesse.
   Bei dem erfindungsgemäßen Sterilisationsverfahren werden sehr hohe Drücke bis etwa 500 MPa angewendet. Aus diesem Grund ist es notwendig, daß die zu sterilisierenden Gegenstände so in der Verpackung angeordnet werden, daß sie bei der Hochdruckbehandlung nicht miteinander verkleben. Bei chirurgischen Fäden ist beispielsweise darauf zu achten, daß diese nicht aufeinanderliegen oder sich überkreuzen. Chirurgische Gegenstände, die einen Hohlkörper bilden, d.h. deren geometrische Form infolge der aufzuwendenden Drücke bruchgefährdet ist, können nach dem erfindungsgemäßen Verfahren dann sterilisiert werden, wenn die Verpackung zusätzlich mit einer inerten Flüssigkeit gefüllt ist. Unter inerten Flüssigkeiten werden erfindunsgemäß solche Flüssigkeiten verstanden, die den hydrolytischen Abbau des resorbierbaren Formkörpers nicht beeinflussen, so z.B. hydrophobe Flüssigkeiten wie Mineralöle, organische Kohlenwasserstoffe - wie z.B. Petrolether. Bevorzugt sind solche Flüssigkeiten, die einen niederen Siedepunkt aufweisen, damit Reste der Flüssigkeit kurz vor der Implantation leicht entfernt werden können. Geeignet sind auch Flüssigkeiten, die mit Wasser (z.B. Kochsalzlösung) mischbar sind und deshalb vor der Implantation abgewaschen werden können. Enthält ein Formkörper zusätzlich einen Wirkstoff, so sind dessen chemische Eigenschaften bei der Auswahl eines geeigneten Lösungsmittels zu berücksichtigen.
   Beispiele für geeignete Polymere sind Homo- und Copolymere auf der Basis von Hydroxycarbonsäuren, wie beispielsweise Polymerisate von Glycolid, Lactid, Methylglycolid, Dimethylglycolid, Polymethylglycolid, Diethylglycolid, Dibutylglycolid, Caprolacton, Valerolacton, Decalacton, Propiolacton, Butyrolacton, Pivalolacton, sowie Polymere auf der Basis von Trioxanon, Dioxanonen (1,3 und 1,4), substituierten Dioxanonen, Trimethylencarbonat, Ethylencarbonat und Propylencarbonat. Als weitere Comonomere sind u.a. die folgenden Verbindungen geeignet: Milchsäure, Glykolsäure, Pentaerythrit, Sorbit, Glycerin, Glycol, Adonit, Xylit, Fructose, Epichlorhydrin, Isopropylmorpholin, Isopropylmethylmorpholindion, β-Propiolsäure, Tetramethylglycolid, β-Butyrolacton, γ-Butyrolacton, Pivalolacton, Hydroxybuttersäuren, Hydroxyisobuttersäuren, Hydroxyvaleriansäuren, Hydroxyisovaleriansäure, Hydroxycapronsäuren, Hydroxyisocapronsäuren, α-Hydroxyα-ethylbuttersäure, α-Hydroxy-α-methylvaleriansäure, α-Hydroxyheptansäure, α-Hydroxyoctansäure, α-Hydroxydecansäure, α-Hydroxytetradecansäure, α-Hydroxystearinsäure.

Geeignet sind ferner Homopolymere aus Lactid, Copolymere aus verschiedenen Lactiden und Copolymere aus Lactiden und Glycolid mit inhärenten Viskositäten zwischen 0.1 und 10 dl/g (Chloroform 25 °C, 0,1 %).

Als Lactid können L-, D-, meso- oder D,L-Lactid oder Gemische davon eingesetzt werden.

Geeignet sind weiterhin Homopolymere aus D,L-Lactid mit einer inhärenten Viskosität von 0.1 bis mindestens 6 dl/g, entsprechend mittleren Molekulargewichten von 2000 bis mindestens 2 Mio.

Ferner sind Copolymere aus L-Lactid und Glycolid mit inhärenten Viskositäten bis mindestens 4.5 dl/g, geeignet, wie auch Copolymere aus D,L-Lactid und Glycolid mit inhärenten Viskositäten von mindestens 2.5 dl/g, weiterhin Copolymere aus ε-Caprolacton mit L-, D-, D,L-Lactid und Glycolid und inhärenten Viskositäten bis mindestens 4 dl/g; ferner z.B.
Poly(L-lactid-co-D,L-lactid),
Poly(L-lactid-co-glycolid),
Poly(D,L-lactid-co-glycolid),
Poly(D,L-lactid-co-meso-lactid),
Poly(meso-lactid-co-glycolid).

Ein weiteres Ziel der vorliegenden Erfindung betrifft ein durch Hochdruckbehandlung sterilisiertes Set bestehend aus einer verschlossenen Verpackung und einem darin eingeschlossen sterilen Formkörper aus einem biologisch abbaubaren Polymer. Im Sinne der Erfindung werden als Verpackung auch Folien, Beutel und Ampullen verstanden. In einer bevorzugten Ausführungsform ist die Verpackung evakuiert und wird erst kurz vor der Anwendung durch Aufreißen belüftet. Zweckmäßigerweise besteht die Verpackung aus einer flexiblen Folie, die heiß verschweißbar ist. Entsprechend geeignete Folien, z.B. aus Polyethylen, Polyvinylchlorid oder anderen bereits genannten Polymeren, sind dem Fachmann bekannt. In einer bevorzugten Ausführungsform besteht die Verpackung aus einem durchsichtigen Material, damit deren Inhalt für den Chirurgen sofort erkennbar ist. Es ist jedoch zusätzlich notwendig, daß die sterilisierten Formkörper ausführlich deklariert werden. Die hierzu notwendigen Etiketten können entweder außen an der Verpackung angebracht werden, z.B. durch Verkleben, oder in einer bevorzugten Ausführungsform mit in die Verpackung eingeschweißt werden.

Dies besitzt den Vorteil, daß innenliegende Etiketten durch die Hochdrucksterilisation, die zumeist - wie bereits erörtert - in einem flüssigen Medium durchgeführt wird, nicht beschädigt oder abgelöst werden können.
Die Verpackung kann dabei so gestaltet sein, daß zwischen dem resorbierbaren Formkörper und dem Etikett keinerlei Verbindung besteht. Dies kann beispielsweise dadurch geschehen, daß durch Heißverschweißung ein geeignetes "Fenster" abgetrennt wird, oder die Folie doppelwandig aufgebaut ist, wobei sich das Etikett zwischen zwei Folien befindet.

Die nachfolgenden Beispiele sollen die Erfindung erläutern, ohne daß sie jedoch auf diese eingeschränkt wird.

Das erfindungsgemäße Verfahren eignet sich auch zur Sterilisation von organischen Wirkstoffen, welche im Trägermaterial gelöst, dispergiert oder mit einem Trägermaterial verbunden sind. Beispielhaft seien genannt: mikroverkapselte Peptide, Peptide oder Proteine, die auf Kieselgel oder anderen Trägern aufgezogen sind.

### Versuche zur Hochdrucksterilisation von Implantaten

Implantatmaterial: DL-Polylactid (Molekulargewicht ca. 300 000) mit ca. 4 % Essigester (Restlösungsmittel, wirkt als Weichmacher).

### Beispiel 1

Experiment: Prüfung der Druckstabilität der Implantate. Es wurden massive Stäbchen (0,8 mm x 0,8 mm x 10 mm), und aufgerollte Folien untersucht.

Prüfkriterien für die Polymerstabilität waren die Glasübergangstemperatur, der Gehalt an Weichmacher (Essigester) und das mittlere Molekulargewicht. Nach 22 Stunden bei 40° C und Drücken von 250 MPa bzw. 300 MPa waren die Werte unverändert. Die Form der Prüflinge blieb erhalten.

### Beispiel 2

### Experiment: Sterilisation von kontaminierten Implantaten (Stäbchen)

Die Implantate wurden kurz in eine Keimmischung (siehe unten) eingetaucht und sofort in Polyethylenfolie eingeschweißt. Diese "Implantat-Briefchen" wurden in einen Schlauch aus Weich-PVC (Innen Durchmesser 3 mm) gesteckt, dessen Enden mit Glasstöpseln verschlossen wurden (um zu vermeiden, daß die Implantate mit dem druckübertragendem Medium Wasser in Berührung kommen, wenn die Folie beschädigt wird).
Die Proben wurden bei 40° C über unterschiedliche Zeiten (2; 6; 24 Stunden) den Drücken von 250 MPa und von 300 MPa ausgesetzt. Parallel dazu wurden je eine Positivkontrolle (Verkeimung, ohne Drucksterilisation) und eine Negativkontrolle (keine Verkeimung, ohne Druck) untersucht.
Zur Sterilitätsprüfung wurden die Implantate in jeweils 10 ml sterile Nährbouillon gegeben und bei 37° C bebrütet. Zeigten die Nährlösungen nach sieben Tagen noch keinerlei Trübung, so waren die Implantate steril.

| | Versuchsbedingung | Keimwachstum nach 7 Tagen |
|---|---|---|
| Negativktr. | 22 Std., unverkeimt, Normaldruck | - |
| Positivktr. | 22 Std., verkeimt, Normaldruck | + (schnelle Trübung) |
| | 2 Std., verkeimt, 250 MPa | + (langsame Trübung) |
| | 6 Std., verkeimt, 250 MPa | + (Trübung nach 3 Tagen) |
| | 22 Std, verkeimt 250 MPa | - |

| b) | | |
|---|---|---|
| Negativk. | 24 Std., unverkeimt, Normaldruck | - |
| Positivk. | 24 Std., verkeimt, Normaldruck | + |
| | 2 Std., verkeimt, 300 MPa | - |
| | 6 Std., verkeimt, 300 MPa | - |
| | 24 Std., verkeimt, 300 MPa | - |
| Bei allen Versuchen 40° C; + bedeutet Keimwachstum - bedeutet kein Keimwachstum | | |

Die Keimmischung enthielt ca 10⁹ Keime pro ml. Sie bestand zu etwa gleichen Teilen aus den folgenden sechs Keimarten.
Streptrococcus faecalis, Aspergillus orycae, Candida albicans, Mikrococcus aureus, Pseudomonas aeruginosa, Escherichia coli.

### Beispiel 3

Schrauben aus Poly(L-lactid) mit inhärenten Viskositäten zwischen 2,1 und 4,0 dl/g (25°C in Cloroform) mit den Abmessungen 4,5 x 10 mm und einem 3° / 30° Sägezahngewinde wurden 22 Stunden bei 40°C mit Drücken von 250 MPa behandelt. Äußere Form und inhärente Viskosität blieben unverändert.

### Beispiel 4

Ein Kondensationsprodukt aus 9 Teilen Glycolsäure und 1 Teil Glycerin mit wachsartiger Konsistenz und den folgenden Analysendaten
Molgewicht (Zahlenmittel) 900 g/mol
Viskosität bei 65 - 70°C 3150 MPa s
Penetrometerwert bei 25°C 5,5 mm
pH-Wert in Wasser nach 2 Min. 4,8
wird in verwendungsfähigen Positionen von 5 g in aluminiumkaschierte Polyethylenbeutel eingefüllt, diese evakuiert und zugeschweißt. Die Beutelchen wurden 22 Stunden in 40°C warmem Wasser bei 250 MPa behandelt. Die danach wiederholte Analyse ergab unveränderte Werte.

## Patentansprüche

1. Verfahren zur Sterilisation von chirurgischen Materialien aus resorbierbaren biologisch abbaubaren Polymeren, dadurch gekennzeichnet, daß das Material für eine vorherbestimmte Zeit Drucken von mindestens 100 MPa unterworfen wird bis eine vollständige Keimabtötung erzielt ist.

2. Verfahren nach Anspruch 1, dadurch gekennzeichnet, daß das chirurgische Material aus einem resorbierbaren Polymeren einen Wirkstoff enthält.

3. Verfahren nach einem der Ansprüche 1 oder 2, dadurch gekennzeichnet, daß das Verfahren in einem Autoklaven in Gegenwart eines Inertgases durchgeführt wird.

4. Verfahren nach einem der Ansprüche 1 oder 2, dadurch gekennzeichnet, daß es in Gegenwart einer inerten Flüssigkeit durchgeführt wird.

5. Verfahren nach einem der Ansprüche 1 bis 4, dadurch gekennzeichnet, daß das chirurgische Material durch eine Verpackung in Form einer Folie oder eines Beutels von dem druckübertragenden Medium getrennt ist.

6. Verfahren nach Anspruch 5, dadurch gekennzeichnet, daß die Verpackung neben dem chirurgischen Material eine pharmakologisch unbedenkliche Flüssigkeit enthält.

7. Verfahren nach Anspruch 6, dadurch gekennzeichnet, daß die pharmakologisch unbedenkliche Flüssigkeit Kochsalzlösung ist.

## Claims

1. A process for the sterilisation of surgical materials made from resorbable biologically degradable polymers characterized in that the material is subjected to pressures of at least 100 MPa for a predetermined period of time until a complete killing of germs is achieved.

2. A process according to claim 1, wherein the surgical material made from a resorbable polymer contains an active substance.

3. A process according to claim 1 or 2, wherein the process is carried out within an autoclave in the presence of an inert gas.

4. A process according to claim 1 or 2, wherein the process is carried out within an autoclave in the presence of an inert liquid.

5. A process according to one of the preceding claims, wherein the surgical material is isolated by a packaging material in the form of a film or a bag from the pressure-transmitting medium.

6. A process according to claim 5, wherein the packaging material contains a pharmacologically unobjectionable fluid besides the surgical material.

7. A process according to claim 6, wherein the pharmacologically unobjectionable fluid is a solution of common salt.

## Revendications

1. Procédé de stérélisation des matériaux de chirurgie en polymère résorbé biodégradable caractérisé par le fait que le matériau soit soumis à une pression d'au moins 100 Mpa, pour une durée déterminée, jusqu'à temps que soit obtenue une destruction totale des germes.

2. Procédé selon la revendication 1,
caractérisé par le fait que le matériau de chirurgie en polymère résorbé contient un agent.

3. Procédé selon une des revendications 1 ou 2,
caractérisé par le fait que le procédé se déroule dans un autoclave en présence d'un gaz inerte.

4. Procédé selon une des revendications 1 ou 2,
caractérisé par le fait qu'il se déroule en présence d'un liquide inerte.

5. Procédé selon une des revendications 1 ou 4,
caractérisé par le fait que le matériau de chirurgie est séparé de la matière transmettant la pression par un emballage sous la forme d'une feuille ou d'une poche.

6. Procédé selon la revendication 5,
caractérisé par le fait que l'emballage contient un liquide sans risque outre le matériau chirurgical.

7. Procédé selon la revendication 6,
caractérisé par le fait que le liquide sans risque, pharmalogique soit une solution salée.
